# EUROPEAN PATENT APPLICATION

(11) **EP 3 187 118 A1**
(43) Date of publication of application: **05.07.2017**
(21) Application number: 17152686.6
(22) Date of filing: 14.07.2009
(51) Int. Cl.: A61B 17/00, A61B 1/018, A61B 1/313, A61B 90/30

(54) **DEVICE TO CLOSE OPENINGS IN BODY TISSUE**

(30) Priority: 21.07.2008 US 129812 P; 19.05.2009 US 453693
(62) Divisional of application: 13003385.5
(71) Applicant: Boston Scientific Scimed, Inc., Maple Grove, MN 55311 (US)
(72) Inventor: Eidenschink, Tracee E.J., Wayzata, MN 55391 (US); Weber, Jan, 6228 GJ Maastricht (NL)
(74) Representative: Vossius & Partner Patentanwälte Rechtsanwälte mbB

(57) **Abstract**

The present invention relates to a device for closing an opening in body tissue, comprising a first end section and a second end section both including one or more anchoring members, and a longitudinal axis. Both end sections are configured to transform in shape from a constrained configuration to an unconstrained configuration, wherein in the constrained configuration the one or more anchoring members are substantially parallel to the longitudinal axis, and in the unconstrained configuration the one or more anchoring members are substantially transverse to the longitudinal axis. The device further comprises a midsection coupled between the end sections, the midsection being self-biased to twist into at least one configuration that substantially prevents the flow of fluid therethrough.

## Description

### CROSS REFERENCE TO RELATED APPLICATIONS

This application claims the benefit of priority of U.S. Provisional Application No. 61/129,812 filed on July 21, 2008, and U.S. Application No. 12/453,693 filed on May 19, 2009, the contents of which are incorporated herein by reference.

### FIELD OF THE INVENTION

Embodiments of the present invention relate to devices to close openings in body tissue. In particular, embodiments of the present invention relate to devices that may be used to close openings in tissue within a body, related methods of closing such openings, and methods of manufacturing such devices.

### BACKGROUND OF THE INVENTION

During recent years, a major drive in surgery has been the development and application of minimally invasive approaches to traditional operations. In general surgery, an emphasis has been on laparoscopic techniques, which can now be applied to the majority of intra-abdominal procedures. The resulting reduction in trauma to the abdominal wall has a positive impact on patients undergoing abdominal operations.

More recently, there has been interest in transluminal endoscopic surgical procedures. In transluminal endoscopic surgery, an endoscope is used to deliberately breach (puncture) the wall of the stomach or other organ to work within the peritoneal cavity. In a transluminal endoscopic surgical procedure, a flexible endoscope (along with the required surgical tools) is inserted into the stomach through a natural anatomic opening. Once the endoscope reaches the stomach, the wall of the stomach is punctured and the endoscope advanced into the abdominal cavity where the remotely controlled surgical tools can be used to perform delicate surgical procedures. When the surgical procedure is completed, the endoscope and the tools are withdrawn through the hole in the stomach and the puncture is dosed.

Although transluminal endoscopic surgery has tremendous potential in reducing trauma associated with surgical procedures, several important developments should be pursued before these procedures can be widely employed. One development is a safe and effective method of closing the puncture in the stomach wall after the endoscope is retracted from the abdominal cavity. Limitations in the mobility of the endoscope and the surgical tools, introduced via a working channel within the endoscope, makes suturing the stomach wall challenging. Therefore, a method of reliability closing a punctured internal body part (such as, a stomach wall) that can be employed using the limited maneuverability offered by an endoscope is required.

### SUMMARY OF THE INVENTION

An embodiment of the invention may include a device for closing an opening in body tissue. The device may include a first end section and a second end section both including one or more anchoring members. Both end sections may be configured to transform in shape from a constrained configuration to an unconstrained configuration. The device may also include a midsection coupled between the end sections. The midsection may have at least one configuration that substantially prevents the flow of fluid therethrough.

Various embodiments of the invention may include one or more of the following aspects: the constrained configuration may correspond to a collapsed shape of the end sections; the unconstrained configuration may correspond to an expanded shape of the end sections; the end sections may be configured to transform from the constrained configuration to the unconstrained configuration when released from a catheter; the one or more anchoring members may be connected together by a base; the base of both end sections may abut the midsection; at least one of the end sections may include a covering material; the covering material may be a fabric; the midsection may be configured to transform from a first configuration to the at least one configuration, wherein the first configuration may correspond to a shape of the midsection when the device is constrained and the at least one configuration may correspond to a shape of the midsection when unconstrained; the midsection may be configured to transform from the first configuration to the at least one configuration by twisting; the midsection may be configured to transform from the first configuration to the at least one configuration when the device is released from a catheter; the end sections may be made of one of an elastic material and a shape memory alloy; at least one of the end sections may substantially resemble a basket; at least one of the end sections may include a plurality of wires and at least some of these wires may be coupled together at opposite ends; the midsection may be made of a fabric; the one or more anchoring members in the constrained configuration may extend along a longitudinal axis; the one or more anchoring members of a distal end section in the constrained configuration may taper towards the longitudinal axis to form a substantially sharp tip; the device may include threads; the device may include an elastic element connecting the first end section and the second end section; the elastic element may be a spring; the elastic element may be configured to twist the midsection to the at least one configuration.

Another embodiment of the invention may include a method for making a device for closing an opening in body tissue. The method includes creating one or more anchoring members coupled to a base, and deforming the one or more anchoring members to form a first end section in a constrained configuration. The constrained configuration may be a shape in which the one or more anchoring members extend along a longitudinal axis of the device. The method may further include forming a midsection between the first end section and a second end section. The midsection may be configured to transform to a configuration that substantially prevents the flow of fluid therethrough.

Various embodiments of the invention may include one or more of the following aspects: creating the one or more anchoring members may include forming the one or more anchoring members from a disk; creating the one or more anchoring members may further include forming a central hole in the base; deforming the one or more anchoring members may include bending the one or more anchoring members in a direction towards the longitudinal axis to form the constrained configuration; forming a midsection may include coupling the midsection to a base of the first end section and a base of the second end section; forming a midsection may include coupling the first end section and the second end section to the midsection such that a plane normal to the longitudinal axis and passing through a center of the midsection forms a plane of reflectional symmetry of the device; the midsection may substantially resemble a hollow tube; forming the midsection may include coupling an end face of the midsection to the first end section and an opposite end face of the midsection to the second end section.

Another embodiment of the invention may include a method of closing an opening in a body tissue. The method may include inserting a catheter containing a closure device at a distal end into a body. The device may include a first end section and a second end section coupled by a midsection. The first end section and the second end section may be constrained configuration within the catheter. The method may also include locating the distal end of the catheter proximate to the opening, and ejecting the first end section out of the catheter such that the first end section transforms from the constrained configuration to an unconstrained configuration on one side of the opening. The method may further include ejecting the second end section out of the catheter to transform the second end section to an unconstrained configuration on an opposite side of the opening, and transforming the midsection to a configuration that substantially closes the opening.

Various embodiments of the invention may include one or more of the following aspects: the constrained configuration may include constraining anchoring members of the first end section and anchoring members of the second end section; ejecting the first end section may include pushing the first end section out of the catheter; transforming the midsection may include transforming the midsection from an open position to a closed position to close the opening; transforming the midsection may include closing a cavity that passes longitudinally through the midsection; transforming the midsection may include twisting the midsection to transform the midsection from the open position to the closed position; ejecting the second end section may include retracting the catheter out of the body to force the second end section out of the catheter; a distal end of the first end section may be configured to form a substantially sharp tip; the method may including creating the opening; creating the opening may include pressing the sharp tip against the body tissue; ejecting the second end section may include rotating the catheter about a longitudinal axis of the catheter.

Another embodiment of the invention may include a device for closing an opening in body tissue. The device may include a tube having opposing end faces, and a plurality of strips separated by slots extending lengthwise between the opposing end faces. The device may also include grooves on the strips positioned transverse to a longitudinal axis of the device. Sections of the strips may be configured to fold along the grooves towards each other when the device is unconstrained.

Various embodiments of the invention may include one or more of the following aspects: the device may further include a covering material disposed on the tube; the covering material may be a fabric; the covering material may be one of a hydrophilic material, a urethane, and a polyester material; the device may be made of shape memory material; the device may have a substantially tubular configuration when constrained within a catheter; the grooves may be located at substantially the same longitudinal location on each strip; the grooves on a first strip may be longitudinally offset from the groove on a second strip.

Another embodiment of the invention may include a method of closing an opening in body tissue. The method may include inserting a catheter containing a closure device at a distal end into a body. The device may be in a constrained configuration within the catheter. The method may also include locating the distal end of the catheter proximate to the opening, and deploying the device proximate the opening such that the device transforms from the constrained configuration to an unconstrained configuration to close the puncture. The transformation may include the device contracting along the longitudinal axis and expanding transverse to the longitudinal axis.

Various embodiments of the invention may include one or more of the following aspects: the unconstrained configuration may be a shape in which the device is substantially planar transverse to the longitudinal axis; deploying the device may include forcing the device out of catheter.

### BRIEF DESCRIPTION OF THE DRAWINGS

The accompanying drawings, which are incorporated in and constitute a part of this specification, illustrate embodiments of the invention and together with the description, serve to explain the principles of the invention.
FIG. 1 is a schematic view of an endoscope performing an exemplary transluminal endoscopic surgical procedure.
FIG. 2 is a schematic view of an exemplary device of the current invention for closing a puncture created during the endoscopic surgical procedure of FIG. 1.
FIG. 3 is an illustration of an exemplary method of making the closure device of FIG. 2.
FIG. 4A is a schematic view of the device of FIG. 2 delivered to a work site internal to the body using a catheter.
FIG. 4B is a cross-sectional view of the catheter of FIG. 4A.
FIG. 5A is a schematic illustration of part of the device being deployed from the catheter of FIG. 4A at the work site.
FIG. 5B is a cross-sectional view of the catheter of FIG. 5A.
FIG. 6 is a schematic view of the kinked midsection of the device of FIG. 2 after being deployed at a work site.
FIG. 7 is a schematic illustration of the catheter of FIG. 4A being withdrawn after the device is deployed at the work site.
FIG. 8 is a schematic illustration of the device of FIG. 2 closing the puncture.
FIGS. 9A-9B are schematic illustrations of other embodiments of a closure device.
FIG. 10 is a schematic illustration of another embodiment of a closure device.
FIG. 11 is a schematic illustration of yet another embodiment of a closure device.
FIG. 12 is a schematic illustration of a further embodiment of a closure device.
FIG. 13A is schematic view of another exemplary closure device of the current invention for closing a puncture created during the endoscopic surgical procedure of FIG. 1.
FIG. 13B is a schematic illustration of the transformation of the device of FIG. 13A to a shape to close the puncture.
FIG. 13C is a schematic illustration of the transformed shape of the device of FIG. 13A closing the puncture.
FIG. 14 is a schematic illustration of the device of FIG. 13A being delivered to a work site internal to the body.
FIG. 15 is schematic view of another exemplary device of the current invention for closing a puncture created during the endoscopic surgical procedure of FIG. 1.
FIGS. 16A-16B are schematic illustrations of an exemplary method of making the device of FIG. 15.
FIG. 16C is a schematic illustration of the device of FIG. 15 loaded on a catheter.
FIGS. 17A-17B are schematic illustrations of an exemplary method of using the device of FIG. 15 to create and close a puncture.
FIGS. 18A-18B are schematic illustrations of an exemplary method of loading the device of FIG. 15 on a catheter.

### DESCRIPTION OF THE EMBODIMENTS

Reference will now be made in detail to exemplary embodiments of the invention, examples of which are illustrated in the accompanying drawings. Wherever possible, the same reference numbers will be used throughout the drawings to refer to the same or like parts. In the description that follows, the closure devices of the invention will be described as being used to close a puncture in body tissue created during endoscopic surgery. However, this illustration is exemplary only, and embodiments of closure devices of the current invention may be used to close any openings in body tissue formed in any manner, even naturally occurring defects and openings.

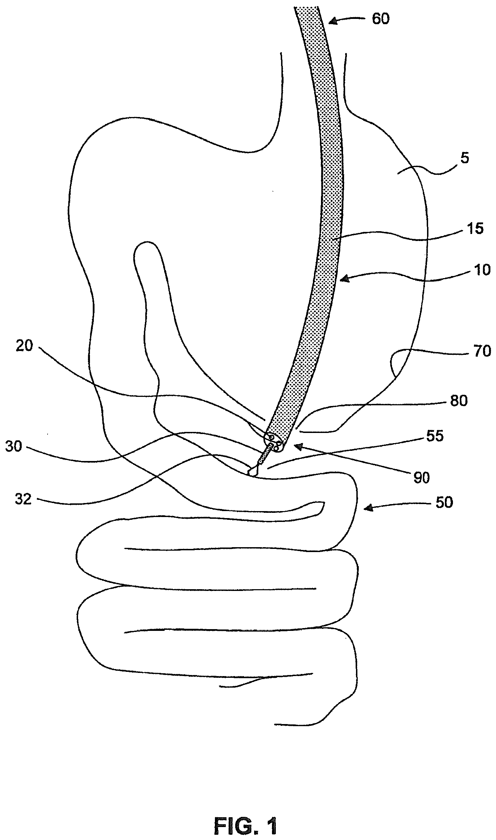

FIG. 1 depicts an exemplary endoscope 10 performing an exemplary transluminal endoscopic surgery. The endoscope 10 may be inserted into the stomach 5 through the esophagus. The endoscope 10 may make a puncture 80 through organ wall 70, pass through the puncture 80, and operate at a work site 55. The work site 55 could include, for instance, part of the small intestine 50. It should be emphasized that although element 10 is described as an endoscope, element 10 could include any device (such as, a catheter, a guide tube, etc.) inserted into the body for diagnostic or therapeutic purposes.

The endoscope 10 may include an elongate member 15 extending between a proximal end 60 and a distal end 90. In the configuration depicted in FIG. 1, the proximal end 60 may include the end of the endoscope 10 external to the body and the distal end 90 may include the end of the endoscope 10 internal to the body. A plurality of lumens 20 may run longitudinally through the endoscope 10. Lumens 20 may extend between the proximal end 60 external to the body and the distal end 90 internal to the body. In some embodiments, the longitudinal axes of the lumens may run along the longitudinal axes of the endoscope 10.

Lumens 20 may include one or more of, among others, an aspiration lumen, irrigation lumen, illumination lumen, viewing lumen, and one or more working lumens. The illumination lumen may include devices at the distal end configured to illuminate work site 55. These devices may include, among others, bulbs, LEDs, fiber optic cables and light guides. The viewing lumen may include devices (such as a CMOS video chip, CCD camera, etc.) at the distal end 90, configured to deliver an image of the work site 55 external to the body. The illumination and the viewing lumens may also include cables that may run from the distal end 90 to the proximal end 60.

The irrigation lumen may be configured to facilitate fluid flow from the proximal end 60 to the distal end 90. In some embodiments, the proximal end 60 of the irrigation lumen may be attached to a source of fluid, and the distal end 90 may be attached to a nozzle to alter fluid flow. The aspiration lumen may be configured to facilitate suction and/or fluid flow through it. In some embodiments, fluid may flow from the proximal end 60 to the work site 55 through the irrigation lumen. The fluid may then be removed from the work site 55 through the aspiration lumen. In some embodiments, the aspiration lumen may also be configured to remove biological material along with fluid from the work site 55. For instance, a tissue sample along with fluid (delivered to the work site 55 via the irrigation lumen) may be extracted out of the body through the aspiration lumen or any other lumen configured for this purpose.

The working lumen may include a hollow cavity configured to deliver an endoscopic instrument 30 to the work site 55. The endoscopic instrument 30 may include a therapeutic or diagnostic tool configured to operate at work site 55, while being remotely controlled from outside the body. The tool may be configured as an end effector 32 that may be attached at the distal end of the endoscopic instrument 30. In general, the working lumen may have any suitable shape, size, and configuration. In some embodiments, the working lumen may have a substantially circular cross-section, while in other embodiments, the working lumen may be keyed or shaped to accept certain devices. For instance, a cross-sectional shape of the working lumen may be configured to pass end effector 32 of endoscopic instrument 30 through it. Some embodiments of the endoscope may include a plurality of working lumens to deliver multiple tools to the work site 55.

In addition to the end effector 32, an endoscopic instrument 30 may also include a mechanism to operate the end effector 32 from outside the body. This mechanism may include linkage that connects the end effector 32 to an actuation device (not shown) at the proximal end. In some embodiments, this linkage may operate the end effector in response to actuation of the actuation device. For example, in some embodiments, the end effector 32 may include forceps with a pair of jaws rotatably coupled to each other. The linkage, in this embodiment, may include a pair of cables, each coupled to a jaw of the forceps at the distal end and to the actuation device at the proximal end. Actuation of the actuation device may move one of the cables relative to the other, causing the jaws of the forceps to open and close.

The end effector 32 may include any medical instrument that may be used in conjunction with a guide tube, catheter, or endoscope 10. In some embodiments, the end effector 32 may be a purely mechanical instrument (for example, biopsy forceps, baskets, graspers, snares, surgical knifes, needles, suturing instruments, etc.), while in others, the end effector 32 may include electrically driven instruments (for instance, heating elements for cauterizing instruments, etc.), or diagnostic elements (such as sensors, lights, etc.).

In the exemplary transluminal endoscopic surgery illustrated in FIG. 1, endoscope 10 may be inserted into the body through a natural anatomic opening (such as, mouth, anus, and vagina, etc.). When the distal end 90 of the endoscope 10 is proximate to an internal surface (such as, an internal organ wall 70), an endoscopic instrument 30 with, for example, an end effector suitable for puncturing organ wall 70, may be delivered to the distal end 90 of the endoscope 10 via the working lumen. The end effector may be used to puncture the organ wall 70. Once organ wall 70 is punctured, the endoscopic tool 30 with the end effector may be withdrawn from the working lumen, and the endoscope 10 inserted in to the organ through the puncture 80. When the distal end 90 of the endoscope 10 is positioned at the desired work site 55 within the organ (such as, for example intestine 50), an endoscopic instrument 30 with an end effector 32 configured to perform a desired task may be delivered to the work site 55 through the working lumen.

The desired operations may be performed at the work site 55 using end effector 32. If more than one tool is required to complete the desired task, other desired end effectors 32 may also be delivered to the work site 55. After completion of the desired operations, endoscope 10 may be retracted from the organ through puncture 80. A closure device 40 of the present invention may now be delivered to the puncture 80 via the working lumen. Device 40 may be configured to close puncture 80. As pointed out earlier, although closure device 40 is described as being used to close intentionally created puncture 80, in general, closure device 40 may be used to close any type of opening in body tissue.

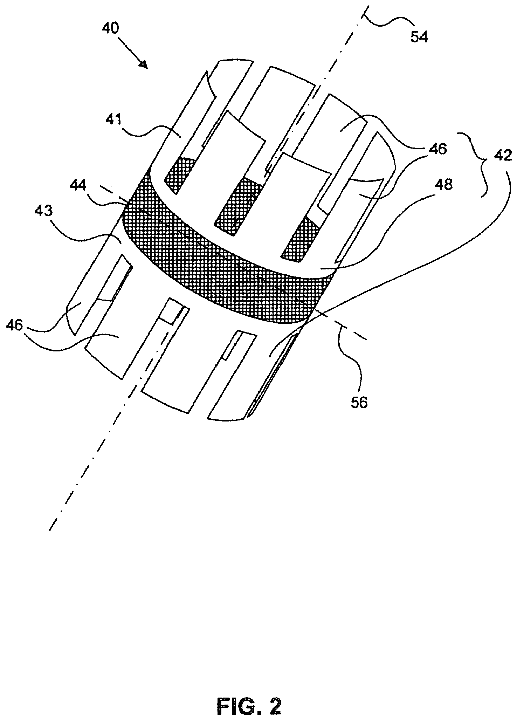

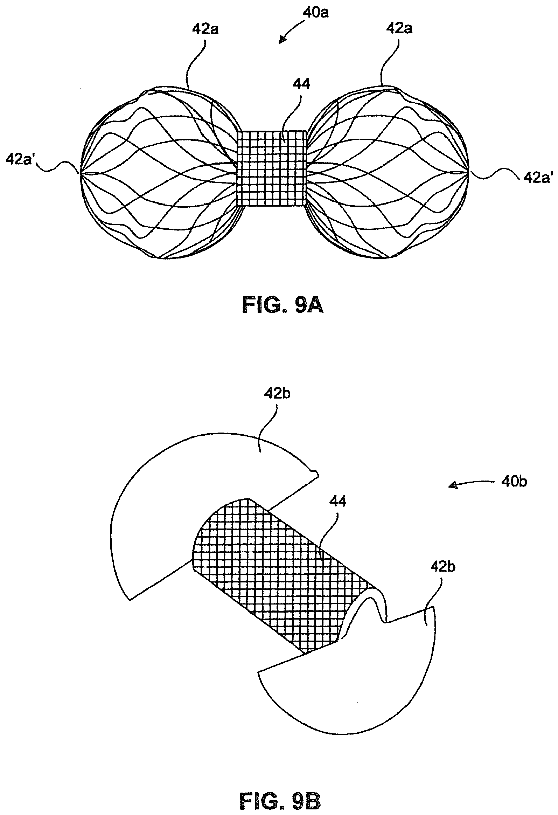

FIG. 2 illustrates a schematic of an embodiment of device 40 that may be delivered to puncture 80. The device 40 may be a multi-component system which includes two end sections 42 coupled together by a midsection 44. The two end sections 42 may include a first end section 41 and a second end section 43 (together referred to as the end sections 42). In the embodiment of the device 40 depicted in FIG. 2, the two end sections 42 are substantially similar and symmetric. That is, in the device 40 depicted in FIG. 2, the first end section 41 is substantially a mirror image of the second end section 43 about a mirror plane 56 normal to a longitudinal axis 54 of device 40. However, it is contemplated that, in some embodiments, the two end sections 42 may not be symmetric, or may be dissimilar. The end sections 42 may include anchoring members 46 connected together by a base section 48. Although FIG. 2 illustrates eight anchoring members 46 extending from base section 48, with each anchoring member 46 being shaped substantially like a petal, it is contemplated that anchoring members 46 may have any shape and configuration. In general, anchoring members 46 may include any number of extensions, and may have any shape (see for instance, FIG. 9B). In some embodiments, in place of discrete anchoring members 46 that extend from base section 48, the anchoring members may be configured differently (such as, for example, baskets depicted in FIG. 9A or mesh-like structures). In some embodiments, end sections 42 may also include a covering material. The covering material may be draped over a surface of the anchoring members 46, to form an umbrella-like geometry. This covering material may be made of a fabric or other suitable biocompatible materials that may collapse when the end section is in the constrained configuration.

End sections 42 may be made of any suitable biocompatible material. And, the material of end sections 42 may have any constitutive behavior (such as, for example, elastic, super-elastic, hyper-elastic, plastic, etc.). In some other embodiments, a shape memory alloy (SMA) may be used for end sections 42. These SMAs may include metallic or polymeric materials. Non-limiting examples of these SMAs may include alloys of titanium-palladium-nickel, nickel-titanium-copper, gold-cadmium, iron-zinc-copper-aluminum, titanium-niobium-aluminum, iron-manganese-silicon, nickel-titanium, nickel-iron-zinc-aluminum, copper-aluminum-iron, titanium-niobium, etc. In some embodiments, the end sections 42 may be made of nitinol.

Each end section 42 may be formed by machining anchoring members 46 from a disk and deforming the anchoring members 46 to a constrained configuration. In some embodiments, this constrained configuration may substantially resemble a tube having a longitudinal axis 54. In some embodiments, the multiple anchoring members 46 may be constrained in the deformed shape by any external means. For example, device 40 may be inserted into a catheter or a tube that keeps end section 42 constrained in the deformed shape. The end sections 42 may be-configured to return to their unconstrained shape when the external force is removed (such as, for instance, when removed from the catheter or the tube). In some embodiments, two end sections 42 may be coupled together with a midsection 44.

Midsection 44 may have any shape configured to couple the two end sections 42 together. In some embodiments, the midsection 44 may include a tubular sleeve. This tubular sleeve may be slotted or grooved to make it more flexible and deformable. Other configurations of midsection 44 (such as a solid slug, etc.) are also contemplated. For instance, in some embodiments, midsection 44 may have a web-like configuration, or possess sealant-like properties. Midsection 44 may be made of a material having a low modulus and/or stiffness to enable midsection 44 to deform easily under compressive force and retain its deformed shape after implantation. In embodiments of midsection 44 with sealant-like properties, these properties may enable the midsection to seal puncture 80.

The midsection 44 may be constructed of a suitable biocompatible material, that may or may not be biodegradable. In some embodiments, midsection 44 may be made of a fabric. Materials that may be used to construct midsection 44 may include, naturally occurring or synthetic, biostable or biodegradable, and may be selected, for example, from the following, among others: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polyether-block co-polyamide polymers (e.g., Pebax.RTM. resins), polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, vinyl aromatic polymers and copolymers such as polystyrenes, styrene-maleic anhydride copolymers, vinyl aromatic-hydrocarbon copolymers including styrene-butadiene copolymers, styrene-ethylene-butylene copolymers (e.g., a polystyrene-polyethylene/butylene-polystyrene (SEBS) copolymer, available as Kraton.RTM. G series polymers), styrene-isoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; ionomers; polyalkyl oxide polymers and copolymers including polyethylene oxides (PEO); polyesters including polyethylene terephthalates, polybutylene terephthalates and aliphatic polyesters such as polymers and copolymers of lactide (which includes lactic acid as well as d-,I- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly (ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen (e.g., collagen IV or V), fibronectin, elastin, chitosan, gelatin, starch, glycosaminoglycans such as hyaluronic acid; as well as blends and further copolymers of the above.

Examples of biodegradable polymers, not necessarily exclusive of those set forth above, may be selected from suitable members of the following, among many others: (a) polyester homopolymers and copolymers such as polyglycolide, poly-L-lactide, poly-D-lactide, poly-D,L-lactide, poly(beta-hydroxybutyrate), poiy-D-gluconate, poly-L-gluconate, poly-D,L-gluconate, poiy(epsilon-caprolactone), poly(delta-valerolactone), poly(p-dioxanone), poly(trimethylene carbonate), poly(lactide-co-glycolide), poly(lactide-co-delta-valerolactone), poly(lactide-co-epsilon-caprolactone), poly(L-lactide-co-beta-malic acid), poly(lactide-co-trimethylene carbonate), poly(glycolide-co-trimethylene carbonate), poly(beta-hydroxybutyrate-co-beta-hydroxyvalerate), poly[1,3-bis(p-carboxyphenoxy)propane-co-sebacic acid], and poly(sebacic acid-co-fumaric acid), among others (b) polyanhydride homopolymers and copolymers such as poly(adipic anhydride), poly(suberic anhydride), poly(sebacic anhydride), poly(dodecanedioic anhydride), poly(maleic anhydride), poly[1,3-bis(p-carboxyphenoxy)methane anhydride], and poly[alpha,omega-bis(p-carboxyphenoxy)alkane anhydrides] such as poly[1,3-bis(p-carboxyphenoxy)propane anhydride] and poly[1,3-bis(p-carboxyphenoxy)hexane anhydride], among others; (c) poly(ortho esters) such as those synthesized by copolymerization of various diketene acetals and diols, and (d) amino acid based homopolymers and copolymers including tyrosine-based polyarylates (e.g., copolymers of a diphenol and a diacid linked by ester bonds, with diphenols selected, for instance, from ethyl, butyl, hexyl, octyl and bezyl esters of desaminotyrosyl-tyrosine and diacids selected, for instance, from succinic, glutaric, adipic, suberic and sebacic acid), tyrosine-based polycarbonates (e.g., copolymers formed by the condensation polymerization of phosgene and a diphenol selected, for instance, from ethyl, butyl, hexyl, octyl and bezyl esters of desaminotyrosyl-tyrosine, among others), and leucine and lysine-based polyester-amides; specific examples of tyrosine based polymers include poly(desaminotyrosyl-tyrosine-ethyl ester adipate) or poly(DTE adipate), poly(desaminotyrosyl-tyrosine hexyl ester succinate) or poly(DTH succinate), poly(desaminotyrosyl-tyrosine ethyl ester carbonate) or poly(DTE carbonate), poly(desaminotyrosyl-tyrosine butyl ester carbonate) or poly(DTB carbonate), poly(desaminotyrosyl-tyrosine hexyl ester carbonate) or poly(DTH carbonate), and poly(desaminotyrosyl-tyrosine octyl ester carbonate) or poly(DTO carbonate), among others. In some embodiments, midsection 44 may be constructed of a polyester fiber such as Dacron®.

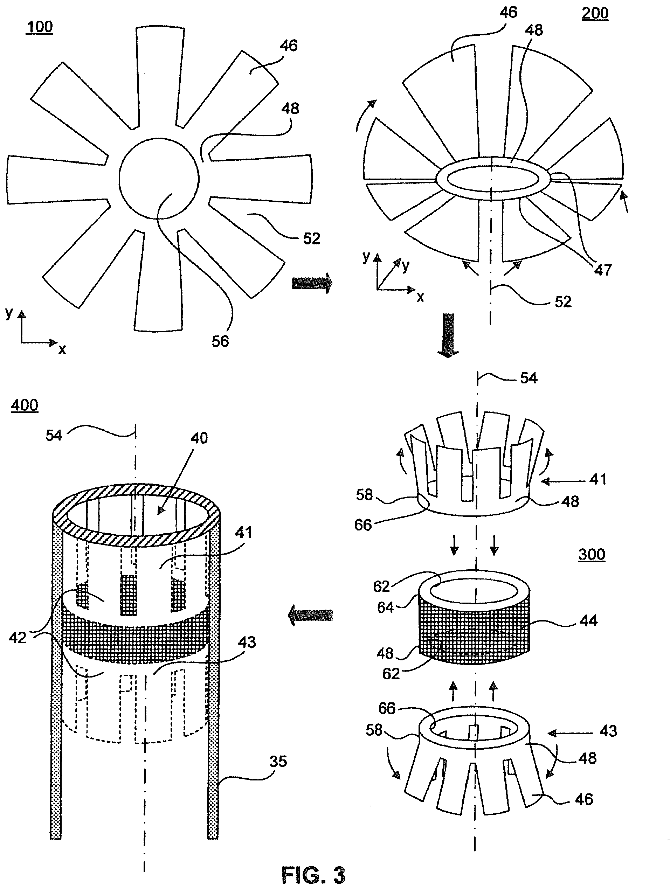

FIG. 3 illustrates an exemplary method of making device 40. End sections 42 may be created from a disk by machining multiple grooves 52 and a central hole 56, in a machining operation 100. The machining operation 100 can include any operation known in the art. In some embodiments, the disk will already include a central hole 56. In these embodiments, the machining operation 100 may only create the grooves 52. Machining grooves 52 from the disk may create anchoring members 46 joined together by a base section 48.

The anchoring members 46 may be folded inwards from an initial configuration to a final configuration in a folding operation 200. The initial configuration of anchoring members 46 may be an unconstrained configuration in which the anchoring members 46 may be transverse to a longitudinal axis 54 extending through central hole 56. The final configuration of the anchoring members 46 may be a constrained configuration in which anchoring members 46 may be substantially parallel to longitudinal axis 54. The folding operation 200 may include deforming the multiple anchoring members 46 inwards such that end section 42, post deformation, substantially resembles a tube. The folding operation 200 may include any operation configured to deform the anchoring members 46 to the constrained configuration. A mechanical force may be applied to deform the anchoring members 46. In some embodiments, the end sections 42 may retain their deformed shape (constrained configuration) after the folding operation 200. In these embodiments, application of energy (for example, heat energy) may cause the anchoring members 46 to return to the unconstrained configuration. In some embodiments, the anchoring members 46 may spring back to the unconstrained configuration when the deforming force is released. In these embodiments, a constraining force may be applied to end sections 42 to constrain the anchoring members 46 in the constrained configuration.

Two end sections 42 may be coupled with midsection 44 in a coupling operation 300, to form the device 40. The two end sections 42 may be coupled to the midsection 44 such that the base sections 48 of both end sections 42 abut the midsection 44. In some embodiments, the two end sections 42 are pressed towards each other with the midsection 44 in the middle in the coupling operation 300. In some embodiments, the end sections 42 may be coupled to the midsection 44 using an adhesive. In some embodiments, end sections 42 and the midsection 44 may be interference fitted. In these embodiments, the diametrical dimensions of the end sections 42 and the midsection 44 may be such that the end section outer circumference 58 may mate with the midsection inner circumference 62. In some embodiments, the end section inner circumference 66 may mate with the midsection outer circumference 64. It is also contemplated that the two end sections 42 may be coupled with a midsection 44 by other means to form device 40.

The device 40 may be inserted into a catheter 35 in the insertion operation 400. The insertion operation may include placing device 40 in the catheter 35 such that the first end section 41 is proximate an end of catheter 35. In the inserted configuration, the longitudinal axes of device 40 and catheter 35 may be substantially collinear, and the end section outer circumference 58 may mate with an internal surface of the catheter 35. In embodiments where a constraining force retains the anchoring members 46 in the constrained configuration, the internal surface of the catheter 35 may provide the constraining force. In some embodiments, one or both of the mating surfaces (of end sections and catheter) may be lubricated prior to inserting device 40 in catheter 35. The insertion operation 400 may include any manual or automated operation.

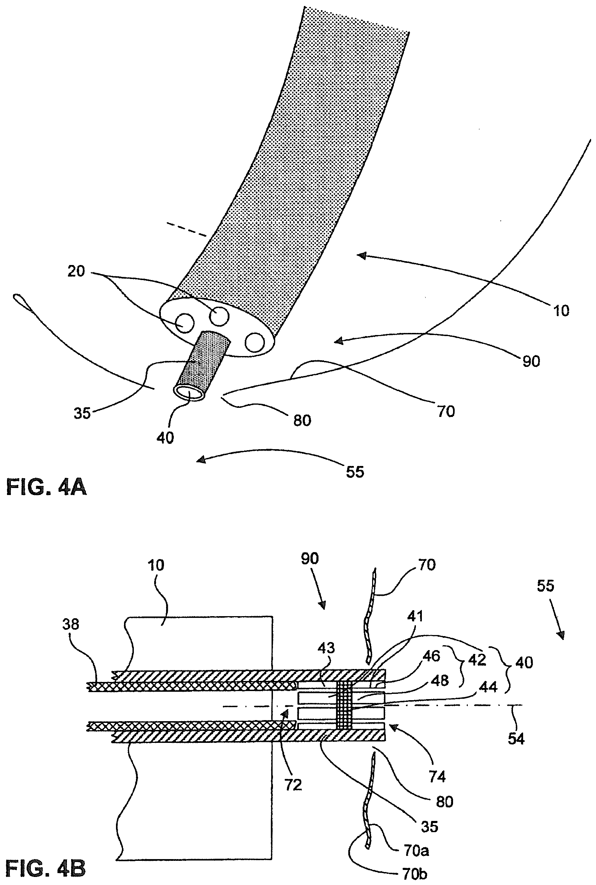

Catheter 35 with the inserted device 40 may be delivered to puncture 80 via a working lumen of endoscope 10. FIG. 4A shows a schematic of the catheter 35 with the inserted device 40 delivered via the working lumen of the endoscope 10. In the schematic depicted in FIG. 4A, the endoscope 10 and the catheter 35 are positioned such that the distal end of the catheter is proximate puncture 80. FIG. 4B shows a cross-sectional view of the endoscope 10 and catheter 35. In the description that follows, reference is made to both FIG. 4A and FIG. 4B. The catheter 35 may be oriented in the working lumen such that the end of the catheter 35 with the device 40 protrudes from the distal end 90 of the endoscope 10, and the first end section 41 of the device 40 is proximate the puncture 80. A push rod 38 may be disposed inside the catheter 35 such that the distal end of the push rod 38 abuts the second end section 43 of the device 40. The push rod 38 may be configured to eject the device 40 out the distal end of the catheter 35. In some embodiments, the push rod 38 may eject only part (for example, first end section 41) of the device 40 out of the catheter 35.

Although in the description above, catheter 35 with device 40 is delivered to puncture 80 through a working lumen of endoscope 10, it is contemplated that other means may be used to deliver device 40 to the puncture 80. For instance, the catheter 35 with the device 40 may be inserted into the body directly through a body cavity. Additionally, push rod 38 (illustrated in FIG. 4B as a hollow tube coaxial with catheter 35) may have other configurations. For instance, push rod 38 can be a solid tube, a rod, a linkage or any other mechanism that may be configured to eject part (or all) of device 40 out the distal end of the catheter 35. In some embodiments, push rod 38 may be configured to conduct temperature or current to the device. It is also contemplated that other means may be utilized to deploy device 40 including, but not limited to, pneumatics, hydraulics, pull wires, and screw mechanisms.

Endoscope 10 may be positioned such that the distal end of the catheter 35 protrudes through puncture 80. While the catheter 35 is thus positioned, the first end section 41 of the device 40 may be ejected out of the catheter 35 by push rod 38 (or any other suitable deployment mechanism). As another example, catheter 35 may be withdrawn relative to device 40 to deploy section 41. In such an embodiment, device 40 may be held stationary by push rod 38 or any other mechanism. Part or all of the midsection 44 may also be ejected along with the first end section 41.

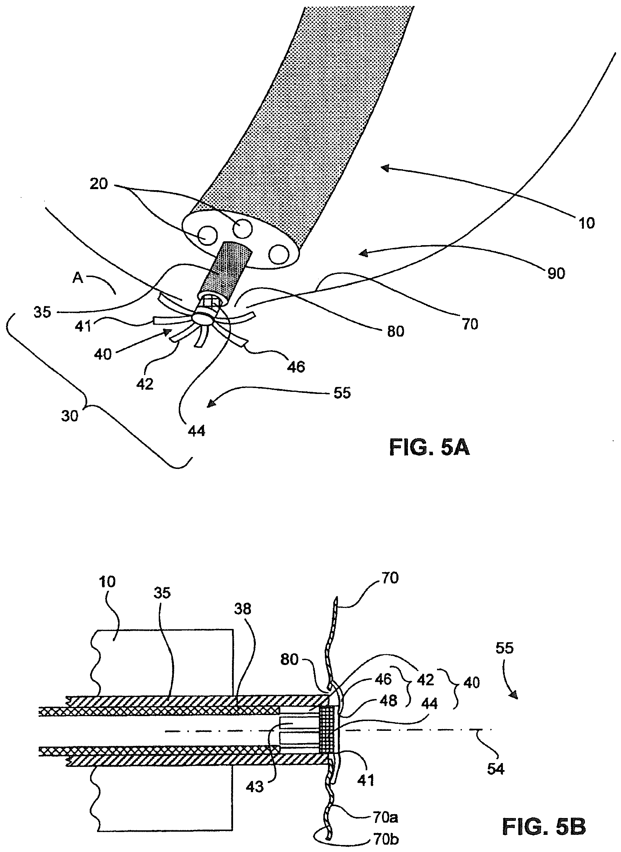

FIG. 5A shows a schematic of the distal end 90 of the endoscope 10 after the first end section 41 is ejected out of the catheter 35. FIG. 5B show a cross-sectional view of the endoscope 10 and catheter 35. In the description that follows, reference is made to both FIG. 5A and FIG. 5B. The anchoring members 46 of the first end section 41 may be configured to unfold when the end section 42 is ejected from of the distal end of the catheter 35. The unfolded anchoring members 46 may press against the outer side 70a of organ wall 70. Although anchoring members 46 are being described as opening and pressing against outer wall 70a, it should be noted that, in general, the side of organ wall 70 that the unfolded anchoring members 46 presses against depends upon the direction of approach of endoscope 10. In the unfolded configuration, the anchoring members 46 may substantially return to the initial unconstrained configuration and form a plane intersecting longitudinal axis 54. In some embodiments, the anchoring members 46 may not completely unfold to the initial unconstrained configuration, but may unfold to a configuration between the initial and final configurations.

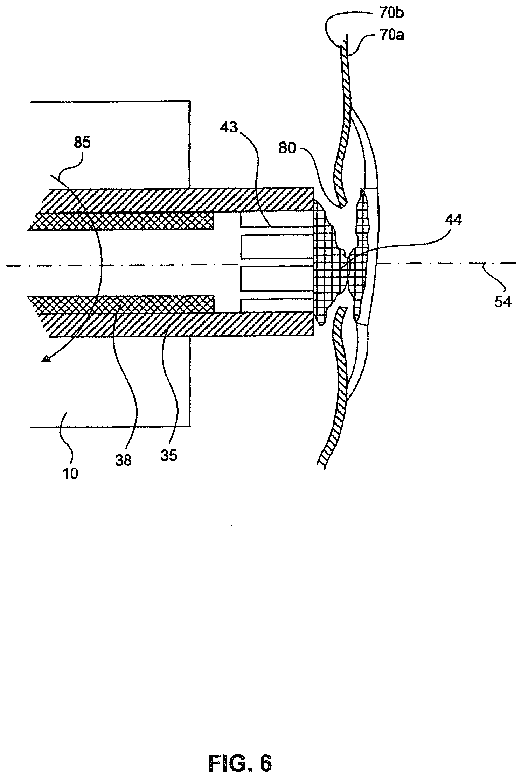

The catheter 35 may now be rotated around the longitudinal axis 54 to twist and kink midsection 44, and the catheter 35 slowly withdrawn from puncture 80. Twisting and kinking midsection 44 may fold parts of the midsection 44 over itself. FIG. 6 shows the kinked midsection 44 of the device 40 after catheter 35 is rotated (depicted by arrow 85) around longitudinal axis 54. In some embodiments, the catheter 35 may be rotated multiple times around the longitudinal axis 54 to completely kink midsection 44. In embodiments where the midsection 44 includes a tubular sleeve, rotating the catheter 35 may kink and close the cavity through the midsection 44. In some embodiments, the step of rotating the catheter 35 may be eliminated. In these embodiments, the catheter 35 may be withdrawn after ejecting the first end section 41. In some embodiments, midsection 44 may be formed of a material or configuration so that it assumes a twisted, kinked, or like configuration after being ejected from catheter 35.

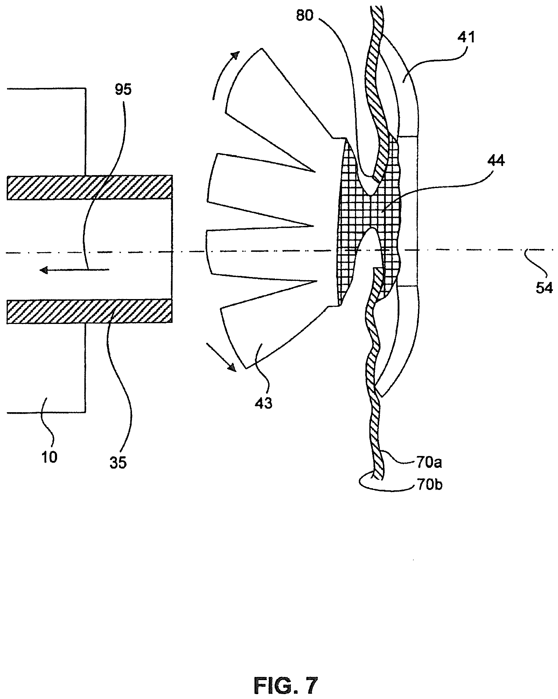

Withdrawing the catheter 35 from puncture 80 may include gently pulling the catheter 35 out of the body through the working lumen of the endoscope 10. FIG. 7 shows a schematic illustrating withdrawing of catheter 35. Withdrawing the catheter 35 (depicted by arrow 95) may drag the midsection 44 (any part still retained within the catheter 35) along with the second end section 43 out of catheter 35. Once out of the catheter 35, the second end section 43 may also unfold on the inner side 70b of the organ wall 70. As with the first end section 41, the second end section 43 may also unfold to the unconstrained configuration. In such an embodiment, midsection 44 may be made of a compliant material that compresses in a longitudinal direction and expands in a transverse direction, when the two end sections 42 press against opposing sides of the organ wall. The expanded midsection 44 may thus help close puncture 80.

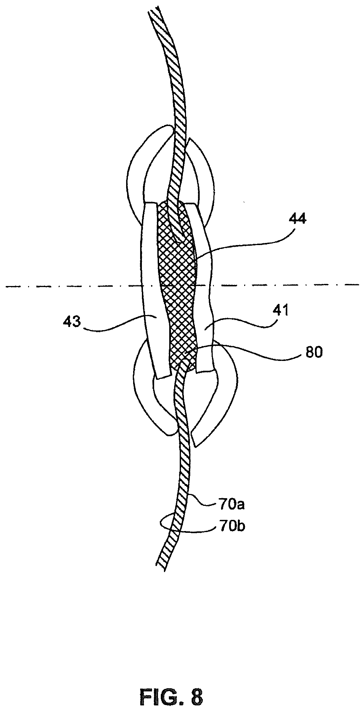

Once device 40 is delivered to puncture 80, the catheter 35 may be withdrawn from endoscope 10, and the endoscope 10 removed from the body. The unfolded end sections 42 of the device 40 along with the midsection 44 may close the puncture 80. FIG. 8 shows an illustration of the device 40 closing the puncture 80. The unfolded end sections 42 may press against the outer and inner side 70a and 70b of the organ wall 70 with the compressed midsection 44 sealing the puncture 80. In embodiments of device 40 with a covering material on end sections 42, the covering material may help in closing puncture 80 by promoting tissue growth around the end sections 42.

Other embodiments of the device may include end sections 42 and midsection 44 configured differently than those described in FIGS. 2-8. FIGS. 9A and 9B illustrate two exemplary embodiments of device 40. FIG. 9A shows a device 40a in the unconstrained configuration. In device 40a of FIG. 9A, end sections 42a are configured as baskets. In the constrained configuration, these basket shaped end sections 42a may collapse to fit within a catheter. Each basket 42a may include a number of wires, threads, or other like members. The members may be arranged in any suitable configuration. For example, each member may be helical, straight, or have another shape. These members of may be joined at a corresponding end 42a'.

FIG. 9B shows another embodiment of device 40b in an unconstrained configuration. In the embodiment of FIG. 9B, end sections 42b are shaped as semicircular extensions. Although end sections 42b are illustrated as being semicircular, in other embodiments, end sections 42b may be of any shape or configuration. For instance, in some embodiments, end sections 42b may be circular or may have any other useful geometry. In the constrained configuration, these end sections 42b may be folder over (as described with reference to FIG. 3) and constrained within a catheter.

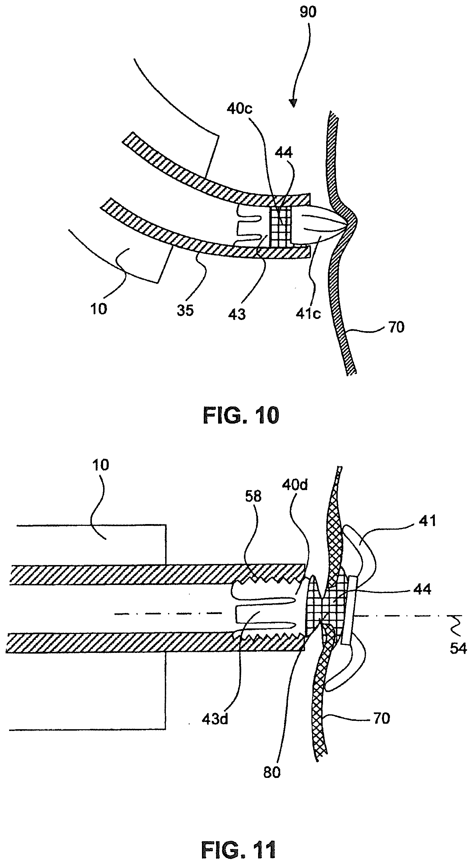

FIG. 10 illustrates an embodiment of the device 40c configured to assist in creating puncture 80 in addition to closing puncture 80. The first end section 41 c in this embodiment may be configured as a sharp tip that functions as a trocar or other puncture creating device. The second end section 43 may also be configured with the sharp tip (similar to the first end section 41), or it may be configured without the sharp tip (as in the embodiments depicted in FIGS. 2-8). In this embodiment, once the distal end 90 of the endoscope 10 is proximate the organ wall 70, the catheter 35 with the inserted device 40c may be delivered to the distal end 90 of the endoscope 10 via the working lumen. The sharp tip of the first end section 41 c may be pressed against the organ wall 70 to create puncture 80. After performing the desired operations within the body, puncture 80 may be closed by deploying device 40c from catheter 35 as described previously. As in previous embodiments, the unfolded end sections along with midsection 44 may seal and close the puncture.

FIG. 11 illustrates another embodiment of device 40d. In this embodiment, the end section outer circumference 58 may be threaded. It is contemplated that the inner surface of the catheter 35 may also be threaded to mate with the threads on the end section outer circumference 58. In this embodiment, ejecting the second end section 43d may involve rotating the catheter 35 around the longitudinal axis 54. Rotation of the catheter 35 around the longitudinal axis 54 may advance second end section 43d out of the catheter 35. The ejected device 40d may then close puncture 80 as described previously. Rotating the catheter 35 around the longitudinal axis 54 may also simultaneously kink and close midsection 44 and eject the second end section 43d. The threads on second end section 43d may also enhance gripping of the organ wall.

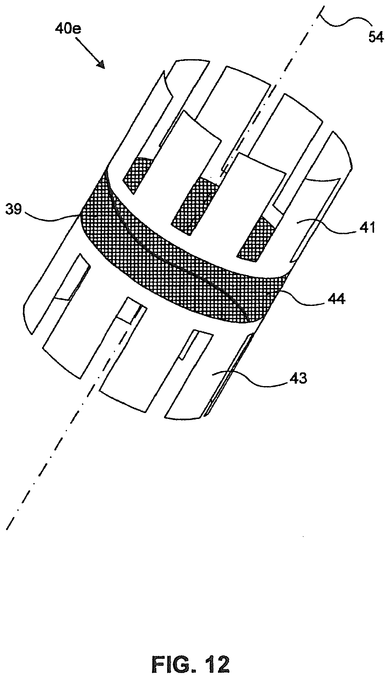

FIG. 12 illustrates another embodiment of the device 40e. In the embodiment depicted in FIG. 12, the first end section 41 and the second end section 43 may be connected together with an elastic element (such as, a wound spring element 39). The spring element 39 may be configured to unwind and rotate end sections 42 with respect to each other (for instance, the first end section 41 with respect to the second end section 43) when released from the catheter 35. This relative rotation of the end sections 42 may kink and collapse the tubular midsection 44 upon itself. In this embodiment, the rotation of the catheter 35 post ejection of the first end section 41 may be eliminated.

In some embodiments of device 40e, the spring 39 may be eliminated and the second end section 43 may itself be biased to rotate the end section when ejected from the catheter 35. This rotation of the second end section 43 may kink and close the midsection 44. In these embodiments, biasing the second end section 43 may be accomplished by constructing the second end section 43 with a shape memory alloy or other materials that may be configured to rotate and unfold to an unconstrained configuration upon ejection from the catheter 35.

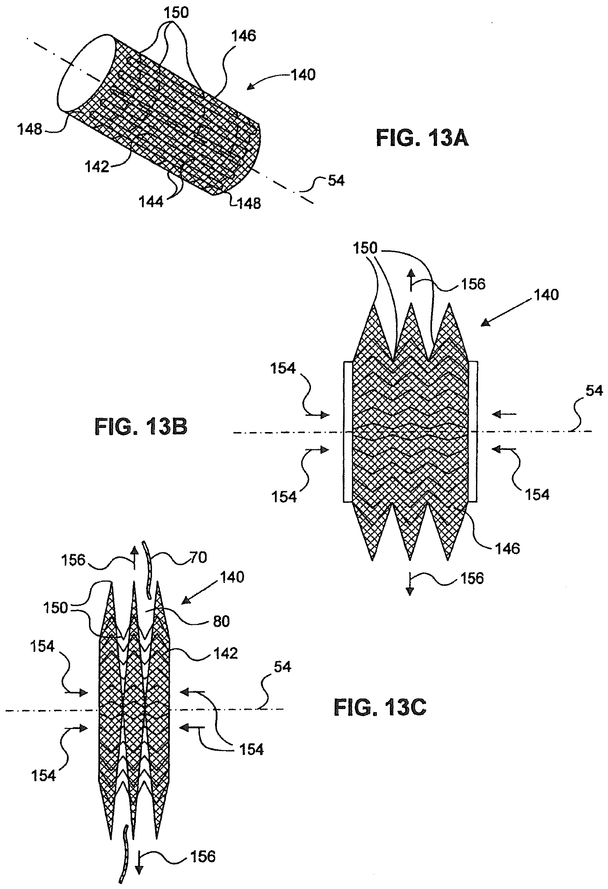

FIGS. 13A-13C illustrate an embodiment of a puncture closing device 140 that closes a puncture 80 by transforming from a constrained configuration to an unconstrained configuration. FIG. 13A illustrates the constrained configuration of the device, and FIG. 13C illustrates the unconstrained configuration. In the constrained configuration, depicted in FIG. 13A, the device 140 may possess a tubular configuration having a longitudinal axis 54. From the constrained configuration, the device 140 may contract in the longitudinal direction (indicated by arrows 154) and expand in the transverse direction (indicated by arrows 156) to transform to the unconstrained configuration (FIG. 13C) through an interim configuration (FIG. 13B). In the unconstrained configuration, the device 140 may possess a substantially planar shape, and may close puncture 80.

In the device 140 of this embodiment, multiple slots 144 may be formed on a tube made of a shape memory alloy. The slots 144 may separate strands 142 of the tube connected by opposite base sections 148. A cylindrical surface of the device 140 may be covered with a covering material 146. In some embodiments, this covering material 146 may include a hydrophilic material. In other embodiments, the covering material 146 may include materials, such as a urethane or a polyester material (for example, Dacron®), that may be configured to have a low stiffness. In some embodiments, covering 146 may be expandable, such as a foam. This foam may bunch up and form a seal at the opening. The device 140 may be subjected to various treatments such that the shape of the device 140 may transform from the constrained configuration to the unconstrained configuration when the device 140 is deployed at the site of the puncture 80.

Treatments on device 140 may include introducing folds 150 on the strands 142 of the device 140. The folds 150 may be created by any mechanical operation. These folds 150 may be configured to act as hinges that may fold different longitudinal sections of the strands 142 on each other. These folds 150 may include a live hinge that separates different sections of the strands 142. In some embodiments, the folds 150 may be created such that the longitudinal location of the folds 150 on each of the strands 142 are substantially the same.

The device 140 may also be subjected to other treatment, such as heat treatment, that may assist device 140 in remembering a configuration or a shape. These heat treatments may assist device 140 to transform from the constrained configuration to the unconstrained configuration when ejected from a catheter 35. To transform from the constrained configuration to the unconstrained configuration, the strands 142 may fold at the folds 150 (as can be seen in FIGS. 13B and 13C).

Although the slots 144 and the strands 142 in the embodiment illustrated in FIGS. 13A-13C, are depicted as substantially straight, any configuration of slots 144 and strands 142 may be used with device 140. For example, in some embodiments, the strands 142 may be curved, tapered, curvilinear, or helically shaped. In some embodiments of device 140, the folds 150 on different strands 142 may be located such that, after transforming to the unconstrained configuration, the folded strands form an interweaving pattern closing the puncture 80. In some such embodiments, the interweaving pattern of the folded strands may make the covering material 146 redundant, and therefore, be eliminated.

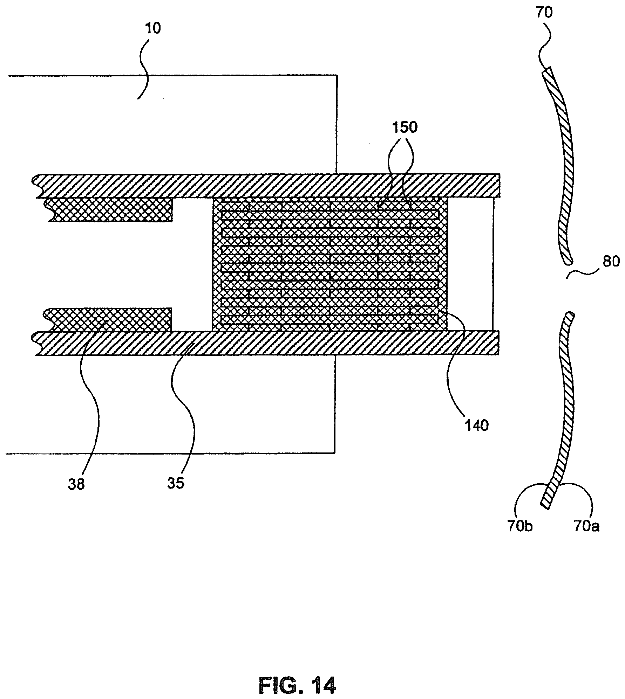

The device 140 of these embodiments may be inserted into a catheter 35 in the constrained configuration and delivered to the site of a puncture 80 through the working lumen of an endoscope, as illustrated in FIG. 14. As described previously, the device 140 may be ejected at the site of puncture 80 using push rod 38. Upon deployment, device 140 may transform to the unconstrained configuration to close puncture 80. In the unconstrained configuration, the covering material 146 may assist in the closing of puncture 80, by hastening tissue growth over the puncture 80.

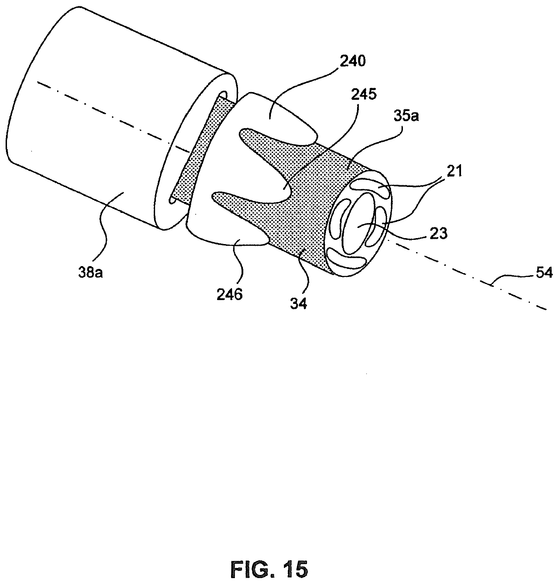

FIG. 15 illustrates another embodiment of a puncture closing device 240 that may be configured to close puncture 80. Unlike the puncture closing devices of previous embodiments that may be delivered to a work site 55 inside a catheter, the puncture closing device 240 of this embodiment may be delivered to the work site 55 external to the catheter. The catheter 35a, in this embodiment, may include a plurality of vacuum lumens 21 along a periphery and a central lumen 23 running longitudinally through the center of the catheter 35a. The device 240 may be loaded on an external surface 34 of the catheter 35a. The device 240 of this embodiment may include a plurality of anchoring members 246 deformed from an unconstrained configuration to a constrained configuration. When ejected from the catheter 35a, the anchoring members 246 may be configured to transform back to the unconstrained configuration. While transforming back to the unconstrained configuration, the tips 245 of the anchoring members 246 may converge on longitudinal axis 54. While converging, sections of organ wall 70 around puncture 80 may be trapped between anchoring members 246, thereby closing the puncture 80. The puncture 80 may be closed by pinching the organ walls 70 around the puncture 80 between the multiple anchoring members 246.

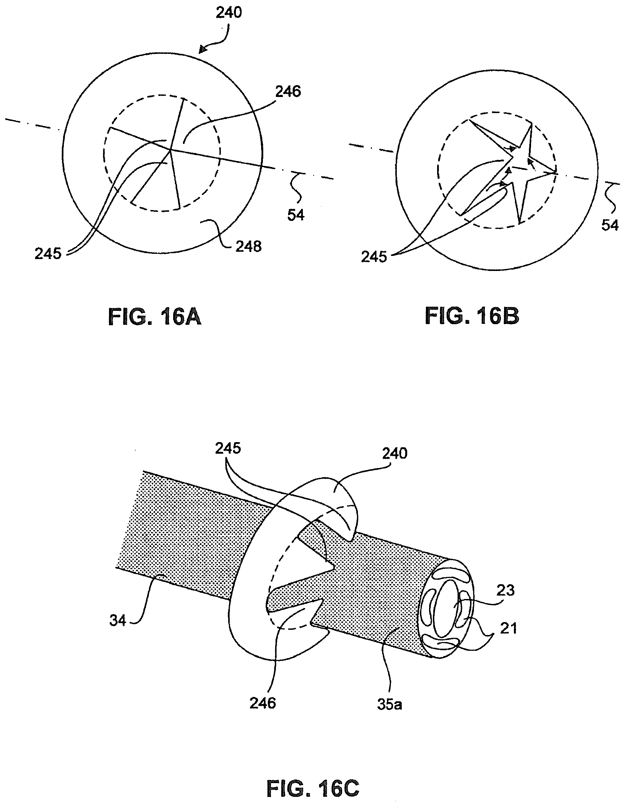

FIGS. 16A-16B illustrate an exemplary method of fabricating device 240. To fabricate device 240, a disk may be machined to form multiple anchoring members 246 that are joined together by a base section 248. FIG. 16A illustrates the unconstrained configuration of the device 240. In the unconstrained configuration, the multiple anchoring members 246 may form flaps with tips 245 that meet at the longitudinal axis 54 that passes through the center of the disk. As illustrated in FIG. 16B, the multiple anchoring members 246 may then be deformed to a constrained configuration. Deforming the anchoring members 246 may include applying a deforming force on the anchoring members 246 to bend the tips 245 of the anchoring members 246 outwards from the longitudinal axis 54. In some embodiments, in the constrained configuration, the anchoring members 246 substantially resemble a frustum of a cone. In some embodiments, the multiple anchoring members 246 may retain the constrained configuration when the deforming force is released. In other embodiments, a constraining force may be applied to the device 240 to keep the anchoring members 246 in the constrained configuration.

FIG. 16C illustrates the constrained configuration of device 240. In the constrained configuration, the device 240 may be loaded on an external surface 34 of the catheter 35a such that the tips 245 of the anchoring members 246 may rest on the external surface 34 of the catheter 35a. In this configuration, interaction of the tips 245 with the external surface 34 may provide the constraining force required to keep the anchoring members 246 in the constrained configuration.

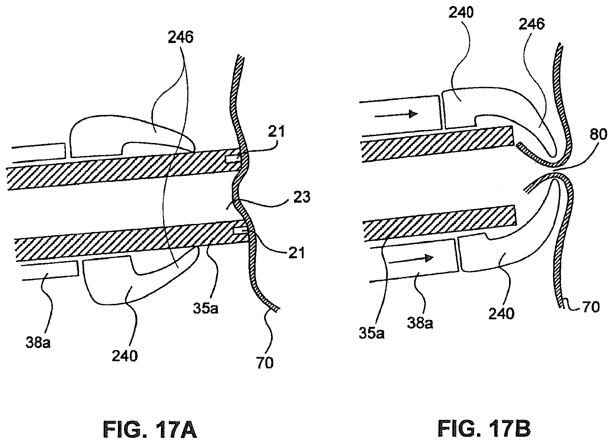

FIGS. 17A and 17B illustrate a method of using device 240 to create and close puncture 80. The catheter 35a may be positioned abutting the region of the organ wall 70 to be punctured. FIG. 17A illustrates a schematic of the catheter 35a positioned abutting the organ wall 70. A vacuum may be applied through the vacuum lumens 21 of the catheter 35a causing part of the organ wall 70 abutting the catheter 35a to attach to the wall of the catheter 35a. A trocar, or other wall puncture device, may be advanced through the central lumen 23 to create puncture 80. In some embodiments, the device 240 may be advanced over the catheter 35a to cause the organ wall 70 around the catheter 35a to stretch, prior to puncturing the organ wall 70. The desired medical procedures may now be performed through the puncture 80.

FIG. 17B illustrates the closing of puncture 80 post completion of the desired medical procedure. The device 240 may be advanced over the catheter 35a using a push rod 38a. The push rod 38a of this embodiment may include a hollow tube coaxial with the catheter 35a, located on the external surface 34 of catheter 35a. However, it is also contemplated that push rod 38a may include other mechanisms, such as a link or a bar, which may advance the device 240 of this embodiment, over the catheter 35a. Advancement of the device 240 may allow the anchoring members 246 to return to the unconstrained configuration. The vacuum through the vacuum lumens 21 may also be deactivated (or decreased) to release the organ wall 70 adhered to the wall of the catheter 35a. The motion of the anchoring members 246 back to the unconstrained configuration may force a part of the organ wall 70 surrounding the puncture 80 to collapse around the puncture 80. The device 240 may be advanced over the catheter 35a until the device 240 slips off the catheter 35a. The part of the organ wall 70 between the anchoring members 246 may now pinch the puncture 80 shut.

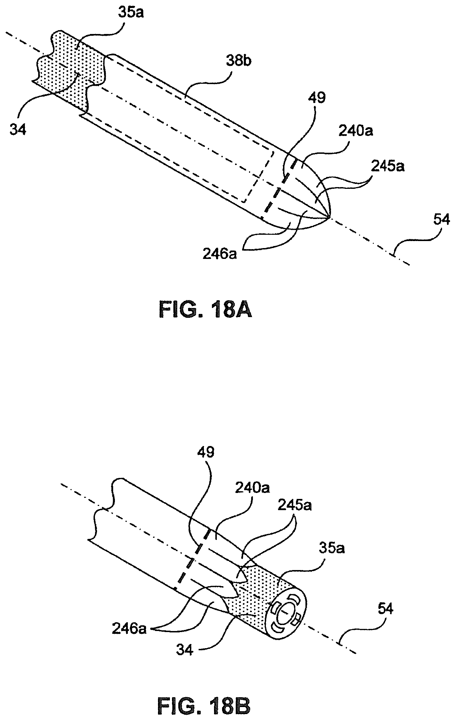

In some embodiments, the device may be part of the push rod. FIGS. 18A and 18B illustrate an embodiment in which the device 240a is part of the push rod 38b. As illustrated in FIG. 18A, the anchoring members 246a may be constructed from a closed end at the distal end of the push rod 38b. As illustrated in FIG. 18B, positioning the push rod 38b on the external surface 34 of the catheter 35a may force the tips 245 of the anchoring members 246a outwards from the longitudinal axis 54 to form the constrained configuration. The external surface 34 of the catheter 35a may further constrain the anchoring members 246a in the constrained configuration. A portion of the push rod distal end that demarcates the device section from the rest of the push rod 38b may also include a region of reduced strength. This reduced strength region may be configured to separate the device 240a from the rest of the push rod 38b. The reduced strength region may include perforations 49, slots, or grooves on the push rod 38b. It is contemplated that the reduced strength region may include other features that are configured to separate on the application of a force. These features may include detachment mechanisms such as hooks, snapping parts, filaments, etc., that may separate the device from the push rod.

Advancing the push rod 38b over the catheter 35a may cause the anchoring members 246a to slip off the distal end of the catheter 35a. The slipping of the anchoring members 246a off the catheter 45a may release the constraining force on the anchoring members 246a causing them to return to their unconstrained configuration. The release of the constraining force combined with the motion of the anchoring members 246a to the unconstrained configuration may provide the force needed to separate the device 240a from the push rod 38b. The motion of the anchoring members 246a back to the unconstrained configuration may also pinch the organ wall 70 around the puncture 80 shut, as in the previous embodiment.

It will be apparent to those skilled in the art that various modifications and variations can be made in the disclosed systems and processes without departing from the scope of the invention. Other embodiments of the invention will be apparent to those skilled in the art from consideration of the specification and practice of the invention disclosed herein. It is intended that the specification and examples be considered as exemplary only, with a true scope of the invention being indicated by the following claims.

### FURTHER DEVICES AND METHODS ARE GIVEN BELOW:

1. A device for closing an opening in body tissue, comprising:
   a first end section and a second end section both including one or more anchoring members, both end sections being configured to transform in shape from a constrained configuration to an unconstrained configuration; and
   a midsection coupled between the end sections, the midsection having at least one configuration that substantially prevents the flow of fluid therethrough.
2. The device of paragraph 1, wherein the constrained configuration corresponds to a collapsed shape of the end sections.
3. The device of paragraph 2, wherein the unconstrained configuration corresponds to an expanded shape of the end sections.
4. The device of paragraph 3, wherein the end sections are configured to transform from the constrained configuration to the unconstrained configuration when released from a catheter.
5. The device of paragraph 1, wherein the one or more anchoring members are connected together by a base.
6. The device of paragraph 5, wherein the base of both end sections abut the midsection.
7. The device of paragraph 1, wherein at least one of the end sections includes a covering material.
8. The device of paragraph 7, wherein the covering material is a fabric.
9. The device of paragraph 1, wherein the midsection is configured to transform from a first configuration to the at least one configuration, wherein the first configuration corresponds to a shape of the midsection when the device is constrained and the at least one configuration corresponds to a shape of the midsection when unconstrained.
10. The device of paragraph 9, wherein the midsection is configured to transform from the first configuration to the at least one configuration by twisting.
11. The device of paragraph 10, wherein the midsection is configured to transform from the first configuration to the at least one configuration when the device is released from a catheter.
12. The device of paragraph 1, wherein the end sections are made of one of an elastic material and a shape memory alloy.
13. The device of paragraph 1, wherein at least one of the end sections substantially resembles a basket.
14. The device of paragraph 1, wherein at least one of the end sections includes a plurality of wires, at least some of the wires being coupled together at opposite ends.
15. The device of paragraph 1, wherein the midsection is made of a fabric.
16. The device of paragraph 1, wherein the one or more anchoring members in the constrained configuration extend along a longitudinal axis.
17. The device of paragraph 1, wherein the one or more anchoring members of a distal end section in the constrained configuration taper towards the longitudinal axis to form a substantially sharp tip.
18. The device of paragraph 1, wherein the device includes threads.
19. The device of paragraph 1, wherein the device further includes an elastic element connecting the first end section and the second end section.
20. The device of paragraph 19, wherein the elastic element is a spring.
21. The device of paragraph 19, wherein the elastic element is configured to twist the midsection to the at least one configuration.
22. A method for making a device for closing an opening in body tissue comprising:
   creating one or more anchoring members coupled to a base;
   deforming the one or more anchoring members to form a first end section in a constrained configuration, the constrained configuration being a shape in which the one or more anchoring members extend along a longitudinal axis of the device; and
   forming a midsection between the first end section and a second end section, the midsection being configured to transform to a configuration that substantially prevents the flow of fluid therethrough.
23. The method of paragraph 22, wherein creating the one or more anchoring members includes forming the one or more anchoring members from a disk.
24. The method of paragraph 22, wherein creating the one or more anchoring members further includes forming a central hole in the base.
25. The method of paragraph 22, wherein deforming the one or more anchoring members includes bending the one or more anchoring members in a direction towards the longitudinal axis to form the constrained configuration.
26. The method of paragraph 22, wherein forming a midsection includes coupling the midsection to a base of the first end section and a base of the second end section.
27. The method of paragraph 22, wherein forming a midsection includes coupling the first end section and the second end section to the midsection such that a plane normal to the longitudinal axis and passing through a center of the midsection forms a plane of reflectional symmetry of the device.
28. The method of paragraph 22, wherein the midsection substantially resembles a hollow tube.
29. The method of paragraph 28, wherein forming a midsection includes coupling an end face of the midsection to the first end section and an opposite end face of the midsection to the second end section.
30. A method of closing an opening in a body tissue comprising:
   inserting a catheter containing a closure device at a distal end into a body, the device including a first end section and a second end section coupled by a midsection, the first end section and the second end section being in a constrained configuration within the catheter;
   locating the distal end of the catheter proximate to the opening;
   ejecting the first end section out of the catheter such that the first end section transforms from the constrained configuration to an unconstrained configuration on one side of the opening;
   ejecting the second end section out of the catheter to transform the second end section to an unconstrained configuration on an opposite side of the opening; and
   transforming the midsection to a configuration that substantially closes the opening.
31. The method of paragraph 30, wherein the constrained configuration includes constraining anchoring members of the first end section and anchoring members of the second end section.
32. The method of paragraph 30, wherein ejecting the first end section includes pushing the first end section out of the catheter.
33. The method of paragraph 30, wherein transforming the midsection includes transforming the midsection from an open position to a closed position to close the opening.
34. The method of paragraph 33, wherein transforming the midsection includes closing a cavity that passes longitudinally through the midsection.
35. The method of paragraph 33, wherein transforming the midsection includes twisting the midsection to transform the midsection from the open position to the closed position.
36. The method of paragraph 30, wherein ejecting the second end section includes retracting the catheter out of the body to force the second end section out of the catheter.
37. The method of paragraph 30, wherein a distal end of the first end section is configured to form a substantially sharp tip.
38. The method of paragraph 37, further including creating the opening.
39. The method of paragraph 38, wherein creating the opening includes pressing the sharp tip against the body tissue.
40. The method of paragraph 30, wherein ejecting the second end section includes rotating the catheter about a longitudinal axis of the catheter.
41. A device for closing an opening in body tissue comprising:
   a tube having opposing end faces;
   a plurality of strips separated by slots extending lengthwise between the opposing end faces; and
   grooves on the strips positioned transverse to a longitudinal axis of the device, wherein sections of the strips are configured to fold along the grooves towards each other when the device is unconstrained.
42. The device of paragraph 41, further including a covering material disposed on the tube.
43. The device of paragraph 42, wherein the covering material is a fabric.
44. The device of paragraph 42, wherein the covering material is one of a hydrophilic material, a urethane, and a polyester material.
45. The device of paragraph 41, wherein the device is made of shape memory material.
46. The device of paragraph 41, wherein the device has a substantially tubular configuration when constrained within a catheter.
47. The device of paragraph 41, wherein the grooves are located at substantially the same longitudinal location on each strip.
48. The device of paragraph 41, wherein the grooves on a first strip are longitudinally offset from the groove on a second strip.
49. A method of closing an opening in body tissue comprising:
   inserting a catheter containing a closure device at a distal end into a body, the device being in a constrained configuration within the catheter;
   locating the distal end of the catheter proximate to the opening; and
   deploying the device proximate the opening such that the device transforms from the constrained configuration to an unconstrained configuration to close the puncture, wherein the transformation includes the device contracting along the longitudinal axis and expanding transverse to the longitudinal axis.
50. The method of paragraph 49, wherein the unconstrained configuration is a shape in which the device is substantially planar transverse to the longitudinal axis.
51. The method of paragraph 49, wherein deploying the device includes forcing the device out of catheter.

## Claims

1. A device (40) for closing an opening in body tissue, comprising:
a first end section (41) and a second end section (43) both including one or more anchoring members (46), and a longitudinal axis (54), both end sections (41, 43) being configured to transform in shape from a constrained configuration to an unconstrained configuration, wherein in the constrained configuration the one or more anchoring members (46) are substantially parallel to the longitudinal axis (54), and in the unconstrained configuration the one or more anchoring members (56) are substantially transverse to the longitudinal axis (54); and
a midsection (44) coupled between the end sections (41, 43), the midsection (44) being self-biased to twist into at least one configuration that substantially prevents the flow of fluid therethrough.

2. The device of claim 1, wherein the constrained configuration corresponds to a collapsed shape of the end sections (41, 43), and the unconstrained configuration corresponds to an expanded shape of the end sections (41, 43), and wherein the end sections (41, 43) are configured to transform from the constrained configuration to the unconstrained configuration when released from a catheter.

3. The device of claim 1, wherein the one or more anchoring members (46) are connected together by a base (48), and the base (48) of both end sections (41, 43) abut the midsection (44).

4. The device of claim 1, wherein at least one of the end sections (41, 43) includes a covering material.

5. The device of claim 4, wherein the covering material is a fabric.

6. The device of claim 1, wherein the midsection (44) is configured to automatically transform from a first configuration to the at least one configuration that substantially prevents the flow of fluid therethrough by automatically twisting when the device is released from a catheter, wherein the first configuration corresponds to a shape of the midsection when the device is constrained and the at least one configuration that substantially prevents the flow of fluid therethrough corresponds to a shape of the midsection (44) when unconstrained.

7. The device of claim 1, wherein the end sections (41, 43) are made of one of an elastic material and a shape memory alloy.

8. The device of claim 1, wherein the one or more anchoring members (46) of a distal end section in the constrained configuration taper towards the longitudinal axis (54) to form a substantially sharp tip.

9. The device of claim 1, wherein the device further includes a spring element (39) connecting the first end section (41) and the second end section (43), and wherein the spring element (39) is inherently biased to cause the midsection (44) to twist into the at least one configuration that substantially prevents the flow of fluid therethrough.
